# EUROPEAN PATENT APPLICATION

(11) **EP 4 837 574 A1**
(43) Date of publication of application: **11.11.2026**
(21) Application number: 24915183.8
(22) Date of filing: 27.12.2024
(51) Int. Cl.: A61K 31/445, A61K 31/4535, C07D 211/46, C07D 409/06, A61P 39/06

(54) **USE OF DNJ OR DERIVATIVE THEREOF IN PREPARING ANTI-SENESCENCE DRUG**

(30) Priority: 03.01.2024 CN 202410013550
(71) Applicant: Zhejiang University, Hangzhou, Zhejiang 310058 (CN)
(72) Inventor: YAN, Qingfeng, Hangzhou, Zhejiang 310058 (CN); LI, Chujun, Hangzhou, Zhejiang 310058 (CN); KONG, Zhengyi, Hangzhou, Zhejiang 310058 (CN); DING, Xue, Hangzhou, Zhejiang 310058 (CN)
(74) Representative: karo IP
(86) International application number: PCT/CN2024/143032
(87) International publication number: WO 2025/145968

(57) **Abstract**

Disclosed in the present invention is use of DNJ or a derivative thereof in preparation of an anti-aging drug. It is found through research in the present invention that DNJ or a derivative thereof has a significant anti-aging effect both *in vitro and in vivo.* The *in vitro* anti-aging effect involves a variety of tissue cells such as endothelial cells, cardiomyocytes, and mesenchymal stem cells, a variety of cell models such as replicative senescence caused by continuous passage or stress-induced senescence induced by H₂O₂, D-galactose, and the like, and a variety of administration modes such as pre-administration, simultaneous administration, or post-modeling administration, all showing that DNJ and a derivative thereof have good anti-aging activity; and an *in vivo* experiment shows that DNJ can significantly delay aging in mice. The use of DNJ or a derivative thereof is innovated in the present invention.

## Description

### TECHNICAL FIELD

The present invention belongs to the technical field of biomedicine, and specifically relates to use of 1-deoxynojirimycin (DNJ) or a derivative thereof in preparation of an anti-aging drug.

### BACKGROUND TECHNOLOGY

Aging is a process of degenerative changes in functions of the body. With increasing age, individuals exhibit a gradual decline in functions of cells, tissues, and organs, and have a significantly increasing risk of various aging-related diseases, such as cardiovascular diseases, neurodegenerative diseases, metabolic diseases, and immune system diseases.

Cellular senescence is a driving factor of aging. Except for reproductive stem cells, normal cells can only undergo a limited number of times of divisions, after which they enter a state of proliferation arrest, namely cellular senescence. The cellular senescence is classified into replicative senescence and stress-induced senescence, wherein the replicative senescence refers to the phenomenon that occurs after a limited number of times of cell divisions and gradual shortening of chromosome telomeres, resulting in the loss of cell differentiation capacity and proliferation arrest; and the stress-induced senescence refers to the phenomenon that occurs after cells respond to pathological stimuli (such as DNA damage and oxidative stress). In senescent cells, the balance of an autophagy-lysosome system is disrupted, leading to an increase in the content of lysosomes and elevated expression of senescence-associated β-galactosidase (SA-β-Gal) in lysosomes. Therefore, the expression of SA-β-Gal is considered one of typical markers of cellular senescence (López-Otín C, et al. Hallmarks of aging: An expanding universe. Cell. 2023; 19; 186(2): 243-278). In addition, although the senescent cells exhibit growth arrest, they still have metabolic activity and secrete numerous bioactive factors, including inflammatory cytokines, chemokines, growth factors, and matrix metalloproteinases, collectively referred to as a senescence-associated secretory phenotype (SASP), which is another important characteristic of the senescent cells. Current studies indicate that SASP includes dozens or even hundreds of bioactive factors. SASP has some differences in composition and content in different tissues and aging models, but in all types of senescent cells generated *in vitro*, the core program of SASP mainly includes pro-inflammatory interleukin-6 (IL-6), CXC chemokine ligand 8 (CXCL8, also known as IL-8), and monocyte chemoattractant protein 1 (MCP1, also known as CCL2) (Di Micco R, et al., Cellular senescence in ageing: from mechanisms to therapeutic opportunities. Nat Rev Mol Cell Biol. 2021, 22(2): 75-95).

The cellular senescence involves a variety of mechanisms, including telomere dysfunction, DNA damage, oncogene activation, mitochondrial dysfunction, chromatin modification, and the like. The mitochondrial dysfunction is closely associated with the induction and maintenance of cellular senescence phenotypes. Mitochondria are not only power sources of cells, but also important sites for regulating cell apoptosis, calcium storage, and signal transduction. Normal mitochondrial functions are essential for maintaining health, and their gradual degeneration leads to senescence phenotypes. The mitochondrial dysfunction in the senescent cells is typically characterized by various mitochondrial functional disorders, including bioenergy imbalance, reduced ATP production, decreased mitochondrial membrane potential, mitochondrial dynamics disorder, impaired autophagy, and the like. As a highly dynamic organelle, the mitochondria, with a balance between fusion and fission, are crucial for maintaining mitochondrial functions and quality control. A significant feature of the cellular senescence is a significant change in mitochondrial dynamics and tissue structure, manifested as elongation or enlargement of the mitochondria and damage to mitochondrial cristae. The loss of cristae in the senescent cells impairs the supply of ATP necessary for maintaining basic cellular functions while decreasing the activity of respiratory complexes and increasing the leakage of electrons and the generation of reactive oxygen species (ROS).

Given critical roles of the senescent cells in a variety of related diseases, great investment has been made in the development of drugs targeting the senescent cells in recent years. Improving the mitochondrial dysfunction in the senescent cells has been considered an idea for the development of anti-aging drugs, and some related clinical trials are in process. For example, β-nicotinamide mononucleotide (NMN) is a naturally occurring nucleotide having bioactivity, is a key intermediate in the synthesis of coenzyme I (NAD⁺), and is considered a supplement for preventing aging; ABT-263 (navitoclax) exerts its anti-aging effect by targeting anti-apoptotic B-cell lymphoma 2 (BCL-2) family proteins located in the mitochondria; Elamipretide, by targeting cardiolipin in mitochondrial inner membranes, has positive effects on a variety of senescence-associated phenotypes in mice; and in addition, some plant-derived polyphenols, triterpenes, and sterol compounds, such as quercetin and rosmarinic acid, have also been found to possess anti-aging activity.

Optic atrophy protein-1 (OPA1) is a dynein located on the mitochondrial inner membranes, which plays an indispensable role in the reconstruction of the mitochondrial cristae and regulates the fusion of the mitochondrial inner membranes to increase an oxidative phosphorylation level. Current studies indicate that OPA1 is a key regulator in the pathophysiology of skeletal muscles and the heart. 1-deoxynojirimycin (DNJ) is a polyhydroxy alkaloid abundantly present in mulberry trees and has a variety of activities, such as anti-diabetic, antioxidant, anti-inflammatory, and anti-obesity activities. Previous work indicates that DNJ promotes the oligomerization of OPA1 by targeting OPA1, thereby reconstructing the mitochondrial cristae and rescuing mitochondrial functions, making it a potential drug for treating mitochondrial hypertrophic cardiomyopathy (Zhuang Q, et al. 1-Deoxynojirimycin promotes cardiac function and rescues mitochondrial cristae in mitochondrial hypertrophic cardiomyopathy. J Clin Invest. 2023; 17; 133(14): e164660.) (Patent No.: 202111036977.5). To improve the bioavailability of DNJ and expand its application, structural optimization is further performed on DNJ to synthesize a series of DNJ derivatives, wherein some compounds, compared with the lead compound DNJ, can better bind to the target protein OPA1, significantly rescue mitochondrial functions, effectively improve cellular physiological states, and exhibit lower cytotoxicity, thus being used for treating diseases related to OPA1polymer formation imbalance, such as hypertrophic cardiomyopathy, deafness, optic nerve atrophy, progressive external ophthalmoplegia and ataxia, progressive myoclonic epilepsy, spastic paresis and intestinal motility disorder, and retinal degeneration (Patent No.: 202211631240.2) (International Application No.: PCT/CN2023/127606).

### SUMMARY OF INVENTION

The present invention further provides use of DNJ or derivatives thereof (DNJ-1, DNJ-3a, DNJ-3b, and DNJ-5a) in preparation of an anti-aging drug.

Use of a 1-deoxynojirimycin compound in preparation of an anti-aging drug is provided, wherein the 1-deoxynojirimycin compound is at least one of the following:
(1) 1-deoxynojirimycin or a pharmaceutically acceptable salt thereof; and (2) a 1-deoxynojirimycin derivative or a pharmaceutically acceptable salt thereof.

Preferably, the 1-deoxynojirimycin derivative is any one of the following:
(1) DNJ-1, having the structure of formula II: 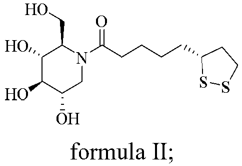
(2) the structure of general formula: 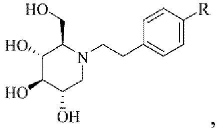 wherein R is selected from -H, -NO₂, and -X, wherein X is F, Cl, Br, or I; and
(3) DNJ-5a, having the structure of formula V:

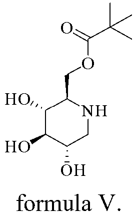

More preferably, when R is selected as -H, the 1-deoxynojirimycin derivative is DNJ-3a, having the structure of formula III:

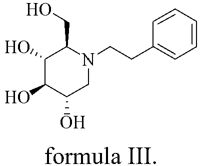

More preferably, when R is selected as -X and X is F, the 1-deoxynojirimycin derivative is DNJ-3b, having the structure of formula IV: 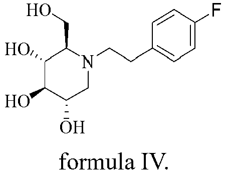
(i) When the 1-deoxynojirimycin derivative is (1), compounds a and b1 are taken and allowed to undergo a condensation reaction in a solvent to obtain the corresponding 1-deoxynojirimycin derivative, wherein the compound a is 1-deoxynojirimycin, and the compound b1 is: 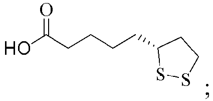
(ii) when the 1-deoxynojirimycin derivative is (4), compounds a and b4 are taken and allowed to undergo a condensation reaction in a solvent to obtain the corresponding 1-deoxynojirimycin derivative, wherein the compound a is 1-deoxynojirimycin, and the compound b4 is: 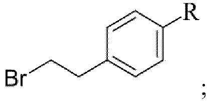 and
(iii) when the 1-deoxynojirimycin derivative is (2), 1-deoxynojirimycin first reacts with benzyl chloroformate to generate an intermediate product 1: 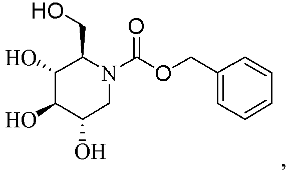
   then, the intermediate product 1 reacts with PivCl to obtain an intermediate product 2: 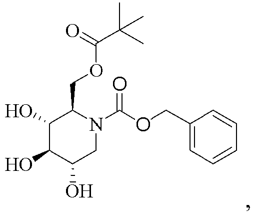
   and finally, the intermediate product 2 is reduced to obtain the 1-deoxynojirimycin derivative.

Preferably, an anti-aging effect of the anti-aging drug is prevention before the occurrence of aging, alleviation during the occurrence of aging, or repair after the occurrence of aging.

Preferably, the anti-aging drug further includes a pharmaceutically acceptable excipient.

Preferably, a dosage form of the anti-aging drug is at least one of a suspension, a granule, a capsule, a powder, a tablet, an emulsion, a dropping pill, an injection, a suppository, an enema, an aerosol, a patch, or a drop.

It is found through research in the present invention that DNJ or a derivative thereof has an anti-aging effect: a variety of tissue-derived cells, such as a human umbilical vein endothelial cell line (Huvec cell line), a rat cardiomyocyte cell line (H9c2 cell line), and human iPSC-derived mesenchymal stem cells (iPSC-MSC), are used to construct stress-induced cellular senescence models by treatment with H₂O₂ and/or galactose (D-gal) and construct replicative cellular senescence models by continuous cell passage, respectively; three different administration modes, including pre-administration, simultaneous administration, and post-modeling administration, are adopted; treatment with NMN is used as a positive control group; the expressions of senescence-associated β-galactosidase (SA-β-Gal) and senescence-associated secretory phenotype (SASP) in the cells are detected to evaluate the anti-aging activity of DNJ or a derivative thereof *in vitro;* and a doxorubicin (Dox)-induced aging mouse model is used to evaluate the anti-aging effect of DNJ *in vivo.* Results indicate that *in vitro,* whether by pre-administration, simultaneous administration, or post-modeling administration, DNJ or a derivative thereof has good anti-aging activity in a variety of tissue cells, such as endothelial cells, cardiomyocytes, and mesenchymal stem cells, as well as under different aging induction modes; and DNJ can significantly delay aging in mice *in vivo*. The use of DNJ or a derivative thereof is innovated in the present invention.

### BRIEF DESCRIPTION OF THE DRAWINGS

FIG. 1 shows a reaction equation of structural formula II.
FIG. 2 shows a mass spectrometry analysis diagram of DNJ-1.
FIG. 3 shows a reaction equation of structural formula III.
FIG. 4 shows a nuclear magnetic resonance (NMR) spectrum of DNJ-3a.
FIG. 5 shows a reaction equation of structural formula IV.
FIG. 6 shows an NMR spectrum of DNJ-3b.
FIG. 7 shows a reaction equation of structural formula VII.
FIG. 8 shows an NMR spectrum of DNJ-5a.
FIG. 9 shows a resistance effect of DNJ or derivatives thereof on H₂O₂-induced senescence in human umbilical vein endothelial cells after pre-administration treatment, wherein A represents SA-β-Gal activity staining images of Huvec cells induced to senescence by H₂O₂ (Sigma) after pre-administration treatment with DNJ or derivatives thereof (DNJ-1, DNJ-3a, DNJ-3b, and DNJ-5a) and a positive control drug (NMN) for 24 hours; and B represents a proportion of SA-β-Gal activity staining-positive cells in Huvec cells after pre-administration treatment, with n = 3, and ****P* < 0.001.
FIG. 10 shows a resistance effect of DNJ or derivatives thereof on senescence in human umbilical vein endothelial cells after simultaneous treatment with H₂O₂, wherein A represents typical SA-β-Gal activity staining images of Huvec cells after simultaneous treatment of DNJ or derivatives thereof (DNJ-1, DNJ-3a, DNJ-3b, and DNJ-5a) and H₂O₂ for 24 hours; and B represents a proportion of SA-β-Gal staining-positive cells in Huvec cells after simultaneous administration treatment, with n = 3, and ****P* < 0.001.
FIG. 11 shows a resistance effect of DNJ or derivatives thereof on senescence in human umbilical vein endothelial cells after induction to senescence by H₂O₂, wherein A represents typical SA-β-Gal activity staining images of Huvec cells treated with DNJ or derivatives thereof (DNJ-1, DNJ-3a, DNJ-3b, and DNJ-5a) after induction by H₂O₂ for 24 hours, respectively; and B represents a proportion of SA-β-Gal staining-positive cells in Huvec cells after simultaneous administration treatment, with n = 3, and ****P* < 0.001.
FIG. 12 shows a resistance effect of DNJ or derivatives thereof on D-galactose-induced senescence in human umbilical vein endothelial cells after pre-administration treatment, wherein A represents typical SA-β-Gal activity staining images of Huvec cells induced to senescence by D-galactose (Sigma) after pre-administration with DNJ or derivatives thereof (DNJ-1, DNJ-3a, DNJ-3b, and DNJ-5a) for 24 hours; and B represents a proportion of SA-β-Gal staining-positive cells in Huvec cells after pre-administration treatment, with n = 3, and ****P* < 0.001.
FIG. 13 shows a resistance effect of DNJ on continuous passage-induced senescence in human umbilical vein endothelial cells, wherein A represents typical SA-β-Gal activity staining images of Huvec cells induced to senescence by continuous passage after treatment with DNJ for 24 hours; and B represents a proportion of SA-β-Gal staining-positive cells in Huvec cells after treatment with DNJ, with n = 12.
FIG. 14 shows a resistance effect of DNJ or derivatives thereof on H₂O₂-induced senescence in rat cardiomyocytes (H9c2 cells) after pre-administration treatment, wherein A represents typical SA-β-Gal activity staining images of H9c2 cells induced to senescence by H₂O₂ after pre-administration with DNJ or derivatives thereof (DNJ-1, DNJ-3a, DNJ-3b, and DNJ-5a) for 24 hours; and B represents a proportion of SA-β-Gal staining-positive cells in H9c2 cells after pre-administration treatment, with n = 3, and ****P* < 0.001.
FIG. 15 shows a resistance effect of DNJ or derivatives thereof on H₂O₂-induced senescence in human iPSC-derived mesenchymal stem cells (iPSC-MSCs) after pre-administration treatment, wherein A represents typical SA-β-Gal activity staining images of iPSC-MSC cells induced to senescence by H₂O₂ after pre-administration with DNJ or derivatives thereof (DNJ-1, DNJ-3a, DNJ-3b, and DNJ-5a) for 24 hours; and B represents a proportion of SA-β-Gal staining-positive cells in iPSC-MSC cells after pre-administration treatment, with n = 3, and ****P* < 0.001.
FIG. 16 shows an improvement effect of DNJ on an inflammatory phenotype of human umbilical vein endothelial cells induced to senescence by H₂O₂ after pre-administration treatment, with n = 4.
FIG. 17 shows an improvement effect of DNJ treatment on an inflammatory phenotype of human umbilical vein endothelial cells induced to senescence by continuous passage, with n = 4.
FIG. 18 shows that DNJ treatment significantly delays aging in mice, with n = 6 in a control group, n = 6 in an aging group, and n = 6 in an administration group.

### DETAILED DESCRIPTION OF THE EMBODIMENTS

For the anti-aging activity of DNJ or derivatives thereof, 3 different cellular senescence models (H₂O₂ stimulation, D-gal stimulation, and continuous cell passage) and 3 different administration modes (pre-administration, simultaneous administration, and post-modeling administration) were selected, and whether DNJ or derivatives thereof could reduce the number of SA-β-Gal staining-positive cells and inhibit the expression of SASP phenotype-associated inflammatory factors was detected in cells derived from a variety of different tissues (human umbilical vein endothelial cells, rat cardiomyocytes, and hiPSC-derived mesenchymal stem cells), respectively. DNJ was continuously fed to *C. elegans* for 30 days to determine whether DNJ could prolong the lifespan of *C. elegans.*

Structural formulas of DNJ and its derivatives DNJ-1, DNJ-3a, DNJ-3b, and DNJ-5a are shown in formulas I to V, respectively.

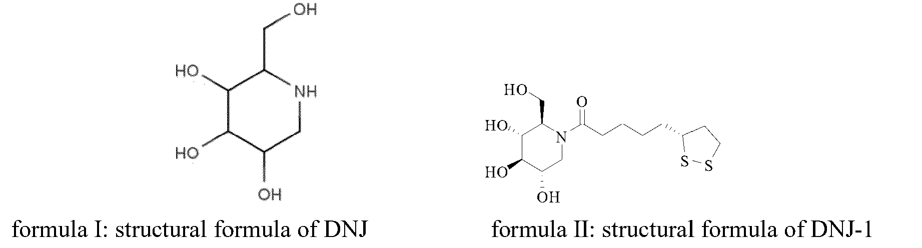

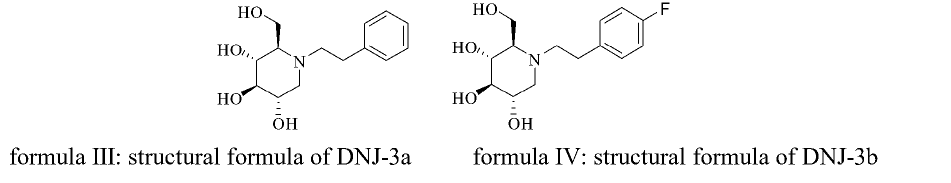

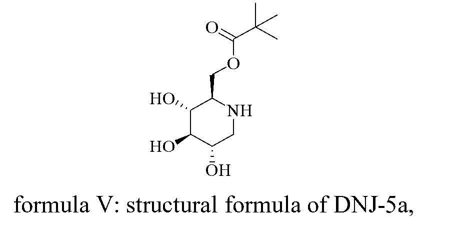

NMN, used as a positive control, is β-nicotinamide mononucleotide.

Preparation of various DNJ derivatives is as follows:
(1) A compound name is DNJ-1; a molecular formula is C₁₄H₂₅NO₅S₂; a structural name is 5-((R)-1,2-dithiolan-3-yl)-1-((2R,3R,4R,5S)-3,4,5-trihydroxy-2-(hydroxymethyl)piperidin-1-yl)p entan-1-one; and a structure is shown as formula II:

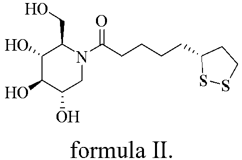

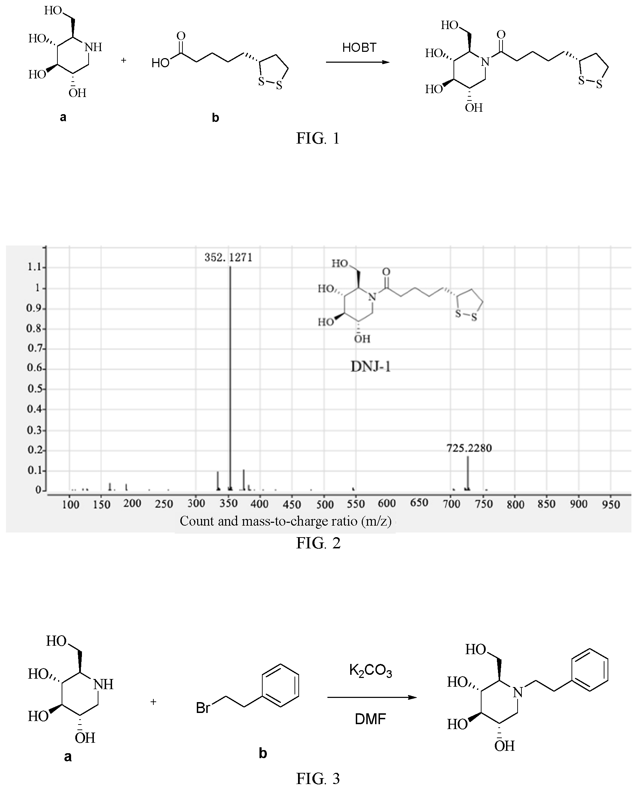

A reaction equation is shown in FIG. 1.

Compounds a (193.97 mg, 1.19 mmol) and b (245 mg, 1.19 mmol) were taken into a round-bottom flask, dichloromethane (DCM, 10 ml) was added as a solvent, the temperature was lowered to 0°C, HOBT (0.177 g, 1.31 mmol) and EDCl (0.251 g, 1.31 mmol) were added, and then, a reaction was carried out at room temperature for 10 hours. After completion of the reaction, the solvent was removed by evaporation, and separation was performed by column chromatography to obtain a product.

FIG. 2 shows a mass spectrometry analysis diagram of DNJ-1.

(2) A compound name is DNJ-3a; a molecular formula is C₁₄H₂₁NO₄; a structural name is (2R,3R,4R,5S)-2-(hydroxymethyl)-1-phenethylpiperidine-3,4,5-triol; and a structure is shown as formula III:

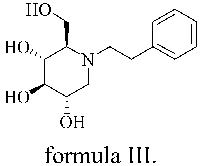

A reaction equation is shown in FIG. 3.

Compound a (163 mg, 1 mmol), compound b (220.79 mg, 1.2 mmol), and potassium carbonate (414 mg, 3 mmol) were taken into a sealed tube, dimethylformamide (DMF, 5 mL) was added as a solvent, and heating was performed to carry out a reaction at 80°C for 4 hours. The reaction was completed according to monitoring by thin-layer chromatography (TLC). The solvent was removed by evaporation, and column chromatography was performed to obtain approximately 80 mg of a product.

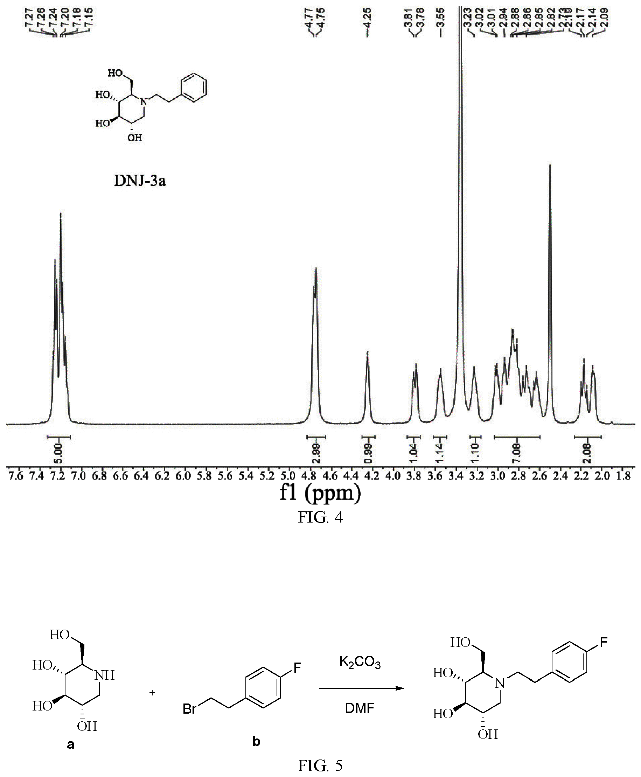

FIG. 4 shows an NMR spectrum of DNJ-3a.

(3) A compound name is DNJ-3b; a molecular formula is C₁₄H₂₀FNO₄; a structural name is (2R,3R,4R,5S)-1-(4-fluorophenethyl)-2-(hydroxymethyl)piperidine-3,4,5-triol; and a structure is shown as formula IV:

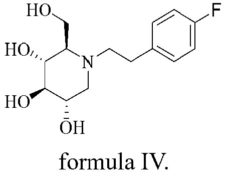

A reaction equation is shown in FIG. 5.

Compound a (163 mg, 1 mmol), compound b (242.38 mg, 1.2 mmol), and potassium carbonate (414 mg, 3 mmol) were weighed and placed in a sealed tube, DMF was used as a solvent, and heating was performed to carry out a reaction at 80°C for 4 hours. The reaction was completed according to monitoring by thin-layer chromatography, the solvent was removed by rotary evaporation, and column chromatography was performed to obtain approximately 70 mg of a product.

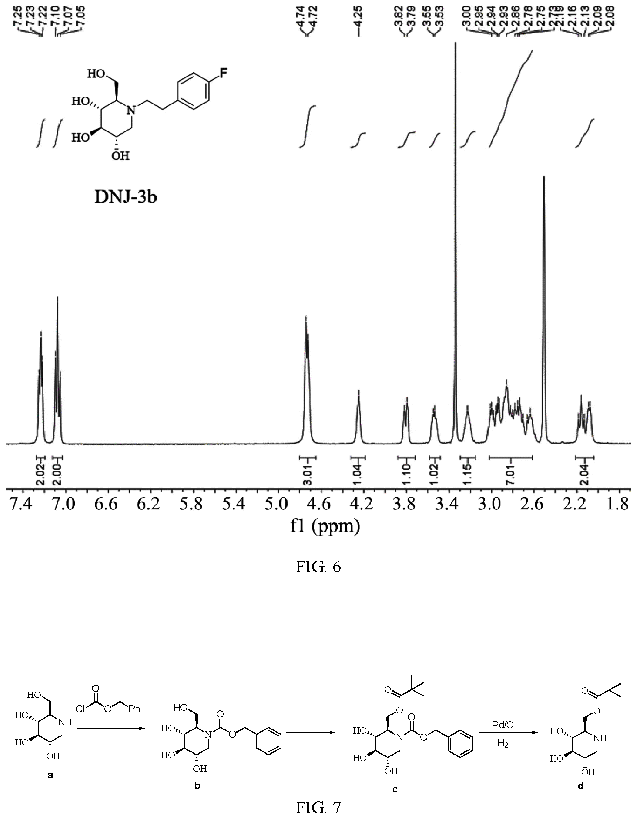

FIG. 6 shows an NMR spectrum of DNJ-3b.

(4) A compound name is DNJ-5a; a molecular formula is C₁₁H₂₁NO₅; a structural name is ((2R,3R,4R,5S)-3,4,5-trihydroxypiperidin-2-yl)methyl pivalate; and a structure is shown as formula VII:

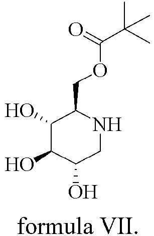

A reaction equation is shown in FIG. 7.

Compound a (115 mg, 0.7 mmol) was weighed and dissolved in a mixture of dioxane and water (1:1, 12 mL), and then, sodium chloride (1.75 equivalents) and benzyl chloroformate (1.54 equivalents) were added and stirred at room temperature for 18 hours. Then, the dioxane was removed by rotary evaporation, extraction was performed with dichloromethane, and an organic layer was retained. Column chromatography was performed to obtain a product b, the compound b (149 mg, 0.5 mmol) was dissolved in pyridine (3 mL), and PivCl (2 times the equivalent) was slowly added at 0°C. PivCl (2 times the equivalents) was further added after 1 hour, and the reaction mixture was diluted with ethyl acetate and methanol after 3 hours. A solvent was removed under reduced pressure, and column chromatography was performed to obtain a compound c.

The compound c (150 mg, 0.39 mmol) was dissolved in MeOH (5 mL), and then, 10% Pd/C (30 mg) was added. The mixture was stirred at room temperature and protected with H₂ overnight. Column chromatography was performed to obtain a compound d.

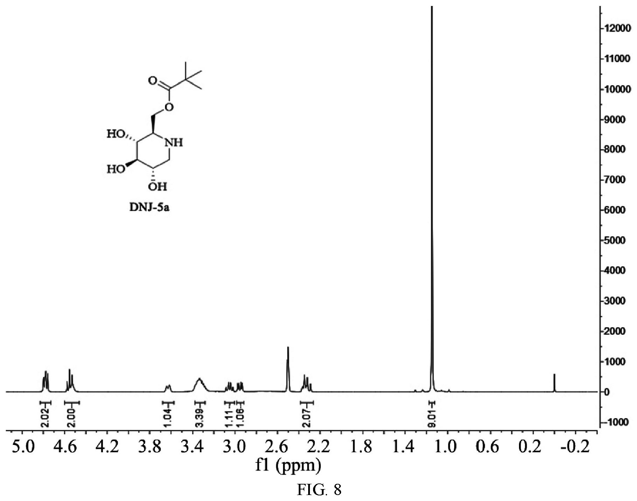

FIG. 8 shows an NMR spectrum of DNJ-5a.

### Example 1

Culture conditions for human umbilical vein endothelial cells (Huvec cells) are as follows:
The Huvec cells were cultured in an ECM culture medium (SclenCell) and passaged and seeded when cultured to a density of 70% to 80% in a culture dish. The cells were washed 1 time with PBS, 0.25% trypsin (Gibco) was added for digestion for 2-3 minutes, and then an ECM culture medium was added to terminate the digestion. The cells were pipetted to be detached, collected into a 15 mL centrifuge tube, and centrifuged at 1,000 rpm for 5 minutes. The supernatant was discarded, a new ECM culture medium was added, and the cells were pipetted uniformly, seeded into a 12-well or 24-well plate (at a density of 70% to 80%), and cultured in a 5% CO₂ incubator at 37°C. After cell adherence, the next operation was performed.

Experimental operation and grouping are as follows:
Experimental grouping: A control group (Control), a modeling group (H₂O₂), a DNJ drug group (H₂O₂ + DNJ, H₂O₂ + DNJ-1, H₂O₂ + DNJ-3a, H₂O₂ + DNJ-3b, H₂O₂ + DNJ-5a), and a positive control drug group (H₂O₂ + NMN) were used.

Working concentrations of each drug and modeling agent: The concentrations of DNJ, DNJ-1, DNJ-3a, DNJ-3b, DNJ-5a, and NMN were all 30 µM; and the concentration of the modeling agent, H₂O₂, was 200 µM.

Experimental operation: After the supernatant was discarded, the cells were washed 3 times with PBS, drug solutions formulated in advance with a culture medium were added, and the blank group and the modeling group were replaced with a blank culture medium without drugs. After culturing for 24 hours, the supernatant was discarded, the cells were washed 3 times with PBS, a blank culture medium was added in the blank group, and a culture medium containing 200 µM H₂O₂ was added in the modeling group and the administration group. After treatment for 24 hours, the cells were subjected to SA-β-Gal staining operation using a cellular senescence β-galactosidase staining kit (Beyotime, C0602).

The workflow for SA-β-Gal staining of the cells is as follows:
For the cells cultured in a 6-well plate, a cell culture solution was sucked away, the cells were washed 1 time with PBS, and 1 mL of an SA-β-Gal staining fixative was added for fixation at room temperature for 15 minutes. For other types of culture plates, the dosages of the fixative and subsequent solutions were used in operation with reference to this ratio. The cell fixative was sucked away, and the cells were washed 3 times with PBS, for 3 minutes each time. PBS was sucked away, and 1 mL of a staining working solution was added to each well. A method for formulating the staining working solution is as follows: A β-galactosidase staining solution A (10 µL), a β-galactosidase staining solution B (10 µL), a β-galactosidase staining solution C (930 µL), and an X-Gal solution (50 µL) were used. The cells were incubated overnight at 37°C, and the 6-well plate was sealed with a parafilm or a plastic wrap to prevent evaporation. Photographing was performed for record using a 20× objective under a microfluidic live cell real-time analysis system (Thermo), cell counting was performed using ImageJ software (National Institutes of Health, v 1.8.0), and ANOVA data analysis of multiple groups (mean ± SD) was performed using Graphpad Prism software (GraphPad Software, v 9.0.0).

Compared with normal cells, senescent cells have the characteristic of high expression of SA-β-Gal. In the cellular senescence β-galactosidase staining kit using X-Gal as a substrate, a deep blue product is generated under the catalysis of senescence-specific β-galactosidase, thereby determining whether the cells are senescent.

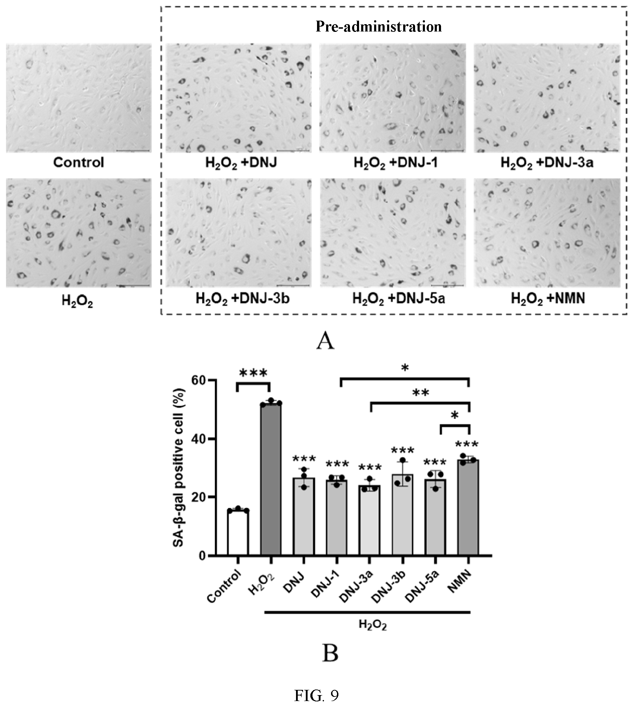

Results are shown in FIG. 9. Compared with the control group, after stimulation with 200 µM H₂O₂ for 24 hours, the number of staining-positive (blue) cells is increased significantly, indicating successful construction of a senescent cell model. After pre-administration treatment with DNJ or its derivatives DNJ-1, DNJ-3a, DNJ-3b, and DNJ-5a for 24 hours, compared with the H₂O₂ group, an increase in the number of senescent cells can be significantly inhibited, and protective effects of DNJ-1, DNJ-3a, and DNJ-5a on senescence are significantly better than that of NMN (with significant differences), indicating that DNJ or its derivatives all have a good preventive effect on senescence.

### Example 2

A basic method refers to Example 1, with differences in the administration method as follows: while inducing by H₂O₂ and constructing a human umbilical vein endothelial cell senescence model, treatment with DNJ or its derivatives was performed separately, and the anti-aging activity of drugs was detected.

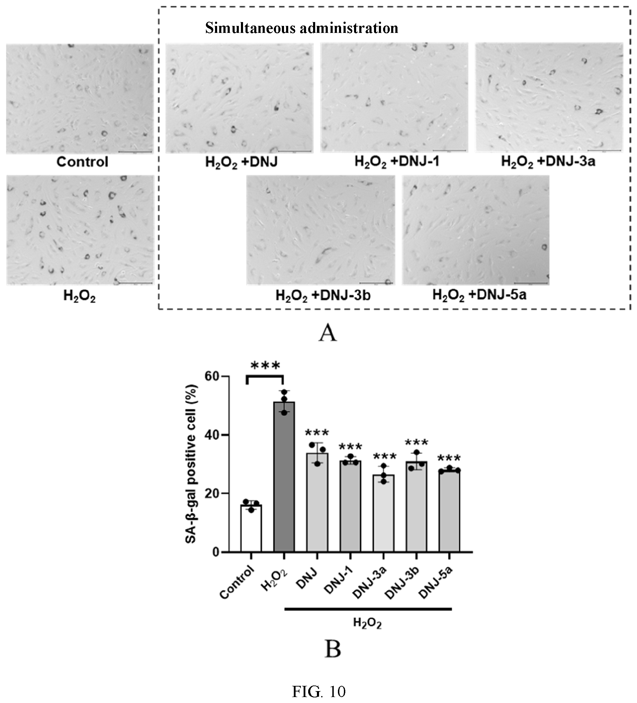

Results are shown in FIG. 10. Staining results show that DNJ or its derivatives DNJ-1, DNJ-3a, DNJ-3b, and DNJ-5a can significantly reduce the production of senescent cells induced by H₂O₂. Statistical analysis shows that the number of SA-β-Gal staining-positive cells is significantly reduced, indicating that DNJ or its derivatives have good anti-aging activity.

### Example 3

A basic method refers to Example 1, with differences in the administration method as follows: after inducing by H₂O₂ and constructing a human umbilical vein endothelial cell senescence model, treatment with DNJ or its derivatives was performed separately, and the anti-aging activity of drugs was detected.

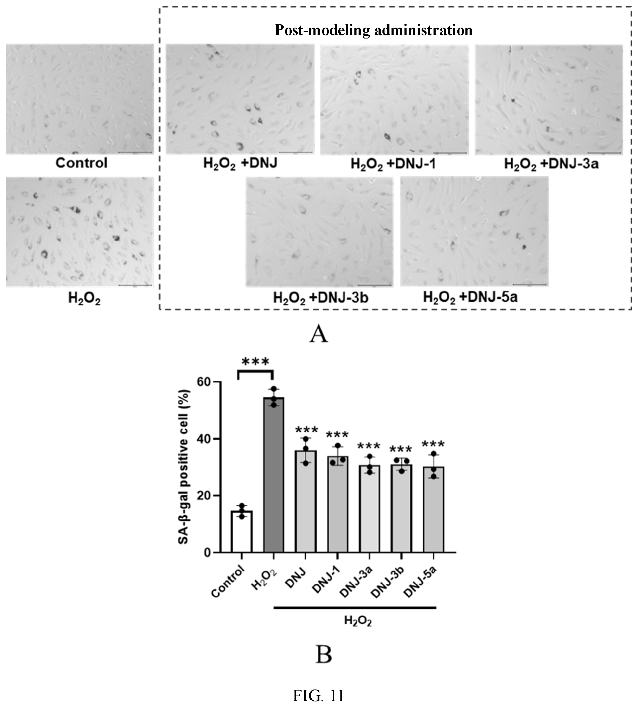

Results are shown in FIG. 11. After stimulation with H₂O₂ for 24 hours to induce senescence, separate addition of DNJ or its derivatives DNJ-1, DNJ-3a, DNJ-3b, and DNJ-5a can significantly reduce the expression of SA-β-Gal and cell senescence, indicating that DNJ or its derivatives have good delaying and therapeutic effects on cellular senescence.

### Example 4

The anti-aging activity of drugs was detected in a stress-induced senescence model of human umbilical vein endothelial cells induced by D-galactose. A basic method refers to Example 1, with differences in the modeling method as follows: a cell senescence model was constructed by stimulation with 40 g/L D-gal for 24 hours.

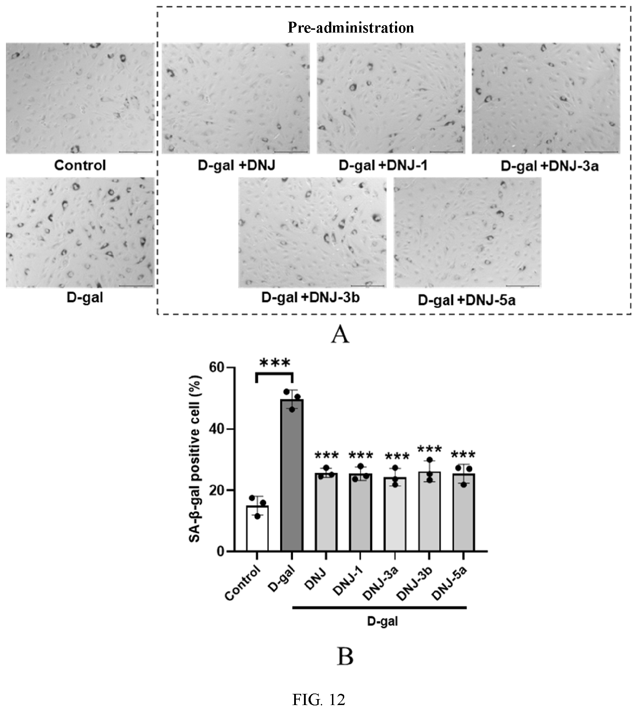

Results are shown in FIG. 12. After stimulation with D-gal for 24 hours, compared with a control group, the number of β-galactosidase staining-positive cells in a D-gal group is increased significantly, confirming successful construction of a senescent cell model. After pre-administration treatment with DNJ or its derivatives DNJ-1, DNJ-3a, DNJ-3b, and DNJ-5a for 24 hours, the number of SA-β-Gal staining-positive cells is significantly reduced, indicating that DNJ or its derivatives have a good anti-aging effect in different senescent cell models during senescence formation.

### Example 5

The anti-aging activity of drugs was detected in a replicative senescence model of human umbilical vein endothelial cells induced by continuous passage. A basic method refers to Example 1, with differences in the modeling method as follows: cell passage was performed when the cells grew to a density of approximately 80%, and the cells of the 4th, 6th, 9th, and 12th generations were used for experiments. Compared with the cells of the 4th generation, the cells of the 6th, 9th, and 12th generations gradually became senescent.

Experimental operation and grouping: Passage operation was performed after the cells grew to a density of 70% to 80%. When passaged to the 4th, 6th, 9th, and 12th generations, the cells were divided into a control group and an administration group, respectively. The control group was cultured in a normal culture medium, and the administration group was added into a culture medium containing 30 µM DNJ for culture. After 24 hours, the cells were subjected to SA-β-Gal staining operation.

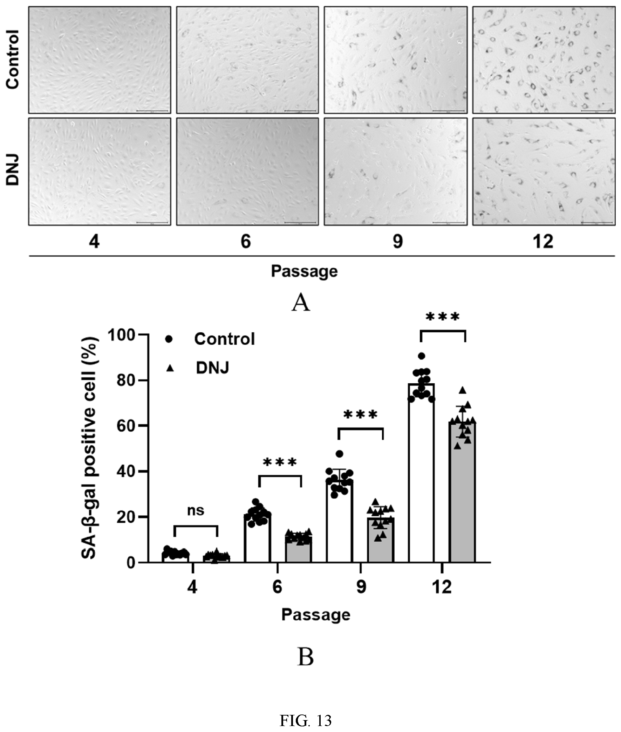

Results are shown in FIG. 13. After continuous passage, compared with the young cells of the 4th generation, the number of β-galactosidase staining-positive cells in the cells of the 6th, 9th, and 12th generations is increased significantly, confirming successful construction of a senescent cell model. Compared with an untreated group, DNJ treatment significantly reduces the number of SA-β-Gal staining-positive cells and the degree of senescence in the cells of the 6th, 9th, and 12th generations, indicating that DNJ has a good anti-aging effect in different senescent cell models during senescence formation.

### Example 6

Culture conditions for rat cardiomyocytes (H9c2 cells) are as follows:
The cells were cultured in a high-glucose DMEM culture medium containing 10% FBS and passaged and seeded when cultured to a density of 70% to 80% in a culture dish. The cells were washed 1 time with PBS, 0.25% trypsin was added for digestion for 2-3 minutes, and a high-glucose DMEM culture medium containing 10% FBS was added to terminate the digestion. The cells were pipetted to be detached, collected into a 15 mL centrifuge tube, and centrifuged at 1,000 rpm for 5 minutes. The supernatant was discarded, a new ECM culture medium was added, and the cells were pipetted uniformly, seeded into a 12-well or 24-well plate (at a density of 70% to 80%), and cultured in a 5% CO₂ incubator at 37°C. After cell adherence, the next operation was performed.

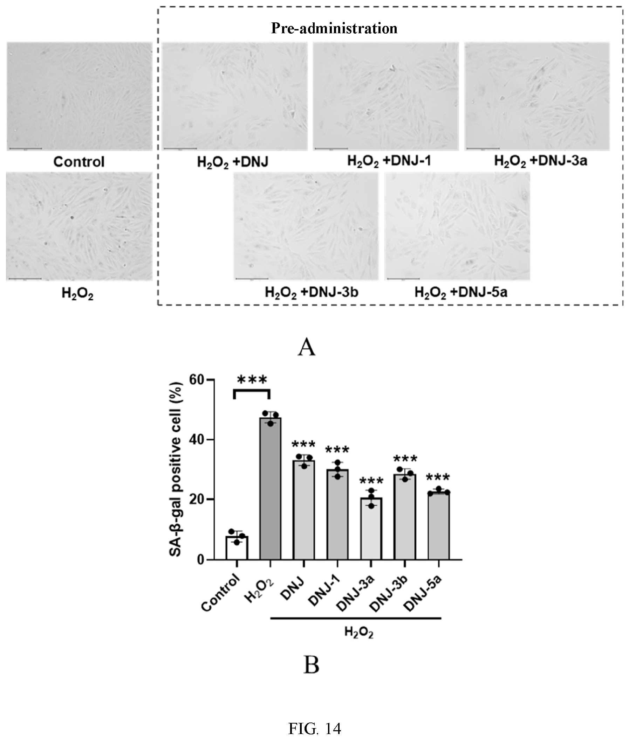

The anti-aging activity of drugs was detected in the H9c2 cells. In the H9c2 cells, stimulation was performed with 400 µM H₂O₂ for 48 hours for modeling. Results are shown in FIG. 14. Compared with a control group, the number of SA-β-Gal staining-positive cells in an H₂O₂ group is significantly increased, indicating successful construction of an H9c2 cell senescence model. The pharmacological activity of DNJ or its derivatives DNJ-1, DNJ-3a, DNJ-3b, and DNJ-5a was detected in the method of pre-administration treatment for 24 hours. DNJ or its derivatives can also reduce the number of SA-β-Gal staining-positive H9c2 cells, indicating that DNJ or its derivatives also have a preventive effect on senescence in cardiomyocytes.

### Example 7

Culture conditions for human iPSC-derived mesenchymal stem cells (iPSC-MSC) are as follows:
The cells were cultured in a MesenCult-ACF Plus Medium (STEMCELL, Cat#05446) and passaged and seeded when cultured to a density of 70% to 80% in a culture dish. After the cells were washed 1 time with DPBS (Gibco), an ACF Enzymatic Dissociation Solution (STEMCELL, Cat#05426) was added for digestion for 2-3 minutes, and then an ACF Enzyme Inhibition Solution (STEMCELL, Cat#05426) was added to terminate the digestion. The cells were pipetted to be detached, collected into a 15 mL centrifuge tube, and centrifuged at 300 G for 8 minutes. The supernatant was discarded, a new MesenCult-ACF Plus Medium was added, and the cells were pipetted uniformly, seeded into a 12-well or 24-well plate (at a density of 70% to 80%), and cultured in a 5% CO₂ incubator at 37°C. After cell adherence, the next operation was performed.

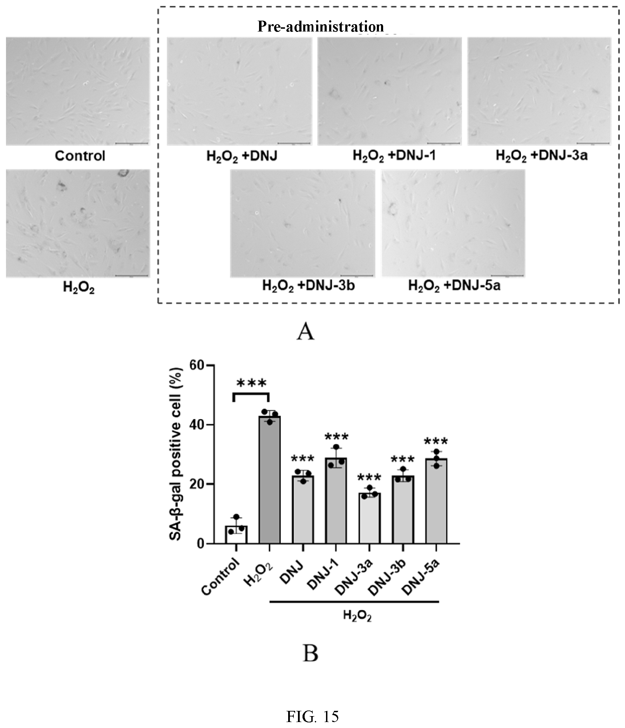

The anti-aging activity of drugs was detected in iPSC-MSC. In iPSC-MSC, stimulation was performed with 40 µM H₂O₂ for 2 hours, followed by replacement with a new culture medium for continued culture for 24 hours to construct a cell senescence model. Results are shown in FIG. 15. Compared with a control group, a significant increase in the number of SA-β-Gal staining-positive cells in an H₂O₂ group confirms successful establishment of an iPSC-MSC senescence model. The pharmacological activity of DNJ or its derivatives was detected in the method of pre-administration treatment for 24 hours. DNJ or its derivatives DNJ-1, DNJ-3a, DNJ-3b, and DNJ-5a can also reduce the number of SA-β-Gal staining-positive iPSC-MSC cells, indicating that DNJ or its derivatives also have an anti-aging effect on mesenchymal stem cells.

### Example 8

The activity of drugs to inhibit a senescence-associated inflammatory phenotype was detected in a stress-induced senescence model of human umbilical vein endothelial cells induced by H₂O₂. A basic method refers to Example 1, with differences in the experimental operation as follows: after the supernatant was discarded, the cells were washed 3 times with PBS, a culture medium containing 30 µM DNJ, formulated in advance in a culture medium, was added, and a blank group and a modeling group were replaced with a blank culture medium without drugs; after culture for 24 hours, the supernatant was discarded, the cells were washed 3 times with PBS, a blank culture medium was added in the blank group, and a culture medium containing 200 µM H₂O₂ was added in the modeling group and an administration group; after treatment for 24 h, RNA samples were extracted using a Steady Pure Quick RNA Extraction Kit (Aikerui), RNA was reverse-transcribed into cDNA using an Evo M-MLV reverse transcription premix kit (Aikerui), and then qRT-PCR reactions were performed using an SYBR^{®} Green Pro Taq HS premix qPCR kit (Aikerui) to detect mRNA expression levels of SASP factors.

The operation procedure for cell RNA extraction is as follows:
A culture solution was sucked out from a culture dish, and the cells were washed once with a 1× PBS buffer solution. The PBS buffer solution was sucked out, 500 µL of a Buffer QLS lysis solution was added, and the culture dish was gently shaken to ensure that the Buffer QLS solution was evenly distributed on cell surfaces. The cells were repeatedly pipetted with a pipette to be detached, and then a homogenate containing the cells was transferred to a centrifuge tube and oscillated at a high speed for uniform mixing or pipetted with a pipette until the homogenate was clear and non-viscous. The homogenate was subjected to standing at room temperature for 2 min. The supernatant was carefully sucked into a new tube (RNase free), and the following operation was performed stepwise:
(1) An equal volume of 100% ethanol was added to the above homogenate and pipetted with a pipette for uniform mixing, and if an obvious viscous substance or precipitate occurred, pipetting was performed several times until the precipitate was dispersed.
(2) The above mixed solution was immediately transferred completely to a Quick RNA Mini Column and centrifuged at 12,000 rpm at room temperature for 2 minutes, and the filtrate was discarded.
(3) 700 µL of Buffer QWB (obtained by mixing Buffer QWB and absolute ethanol at a volume ratio of 3:7) was added to the Quick RNA Mini Column and centrifuged at 12,000 rpm at room temperature for 1 minute, and the filtrate was discarded.
(4) The Quick RNA Mini Column, as an adsorption column, was removed vertically to prevent a column head of the adsorption column from getting in contact with a wall of a collection tube, placed onto a new 2.0 ml Collection Tube, and centrifuged at 12,000 rpm at room temperature for 2 min.
(5) The Quick RNA Mini Column, as the adsorption column, was placed onto a new RNase Free Tube, 50 µl to 200 ml of RNase Free Water was added to the center of an adsorption column membrane, and standing was performed at room temperature for 3 minutes to dissolve RNA, followed by centrifugation at 12,000 rpm at room temperature for 2 minutes to elute RNA. RNA concentrations of samples were detected using a Nano-100 micro-spectrophotometer, RNA purities were detected based on absorbance at 260/280 nm, and the samples were finally stored at -80°C.

The operation procedure for reverse transcription and qRT-PCR reactions of cell RNA samples is as follows:
(1) Removal of genomic DNA. According to the corresponding RNA concentrations, the required volumes of RNase free water and RNA were calculated. 2 µL of a 5× gDNA Clean Reaction Mix and the corresponding volumes of RNase free water and RNA were added to a 200 µL EP tube to ensure a total liquid volume of 10 µL. The EP tube was placed in a reverse transcription instrument to undergo a genomic DNA removal reaction according to the following procedure: 42°C, and cooling to 4°C after 2 minutes.
(2) Reverse transcription reaction. The reaction solution in the step (1) was removed, and 4 µL of a 5× Evo M-MLV RT Reaction Mix and 6 µL of RNase free water were added respectively to ensure a total liquid volume of 20 µL. The EP tube was placed in a reverse transcription instrument to undergo denaturation and annealing reactions according to the following procedure: 37°C for 15 minutes, 85°C for 5 sec, and cooling to 4°C. The samples were used for qRT-PCR experiments.

qRT-PCR reactions were performed using an SYBR^{®} Green Pro Taq HS premix qPCR kit (Aikerui). 2 µL of cDNA solutions of the corresponding samples, 0.08 µL of forward and reverse primers, 5 µL of a 2× SYBR^{®} Green Pro Taq HS Premix, and 2.92 µL of RNase free water were respectively sucked into new EP tubes to ensure that the total liquid volume for each replicate was 10 µL (sequences of the forward and reverse primers for target genes are shown in Table 1). A detection procedure was set as follows: pre-denaturation (95°C, 30 seconds; 95°C, 5 seconds), annealing (60°C, 30 seconds), 40 cycles, and on-machine detection.

### Data analysis:

(1) A Ct value of a control group (H1) was subtracted from a sample group to obtain ΔCt.
(2) A Ct value of a reference gene group (β-actin) was subtracted from a target gene group to obtain ΔΔCt.
(3) A relative expression amount of a target gene was calculated as mRNA expression (fold change, FC) = 2^ - (ΔΔCt).

**Table 1 Primer sequence**

| Gene | Primer sequence | Gene | Primer sequence |
|---|---|---|---|
| IL6 | F: ACTCACCTCTTCAGAACGAA TTG | IL1β | F: ATGATGGCTTATTACAGTGG CAA |
| | R: CCATCTTTGGAAGGTTCAGG TTG | | R: GTCGGAGATTCGTAGCTGG A |
| IL8 | F: ACTGAGAGTGATTGAGAGT GGAC | CCL2 | F: CAGCCAGATGCAATCAATG CC |
| | R: AACCCTCTGCACCCAGTTTT C | | R: TGGAATCCTGAACCCACTT CT |
| IL1 α | F: AGATGCCTGAGATACCCAAA ACC | β-ACTI N | F: CATGTACGTTGCTATCCAGG C |
| | R: | | R: |
| | CCAAGCACACCCAGTAGTCT | | CTCCTTAATGTCACGCACGA T |

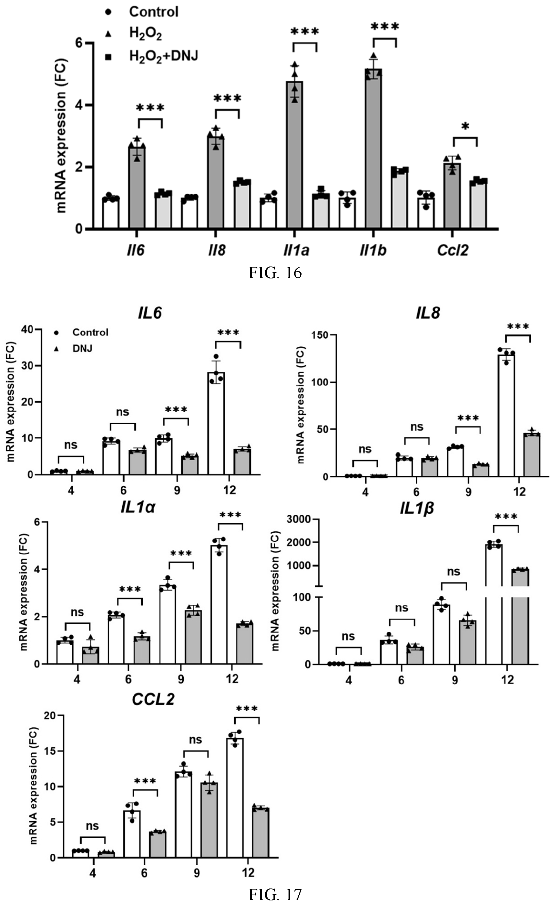

Compared with normal cells, senescent cells have the characteristic of high expression of SASP factors. The core program of SASP mainly includes pro-inflammatory interleukin-6 (IL-6), CXC chemokine ligand 8 (CXCL8, also known as IL-8), and monocyte chemoattractant protein 1 (MCP1, also known as CCL2). Results are shown in FIG. 16. Compared with a control group, after stimulation with 200 µM H₂O₂ for 24 hours, the mRNA expression levels of SASP factors are significantly increased. After pre-administration treatment with DNJ for 24 hours, compared with an H₂O₂ group, an increase in the mRNA expression of SASP factors can be significantly inhibited, indicating that DNJ has the effect of improving the SASP phenotype of senescent cells.

### Example 9

The activity of drugs to inhibit a senescence-associated inflammatory phenotype was detected in a replicative senescence model of human umbilical vein endothelial cells induced by continuous passage. A modeling method and experimental grouping refer to Example 8, and basic operation refers to Example 9. Results are shown in FIG. 17. Compared with young cells of the 4th generation, the mRNA expression levels of SASP factors in senescent cells of the 6th, 9th, and 12th generations are significantly increased. After separate treatment with DNJ for 24 hours, compared with a control group, an increase in the mRNA expression of SASP factors can be significantly inhibited, indicating that DNJ has the effect of improving the SASP phenotype of senescent cells.

### Example 10

An effect of drugs in delaying aging *in vivo* was detected using a mouse model. Male C57BL/6J mice were selected in the experiment, and randomly divided into a normal group (Control), an aging group (Aging), and an administration group (Aging + DNJ). Dox was intraperitoneally injected into the mice continually for one week to construct an aging mouse model; and the administration group was treated with DNJ simultaneously during the construction of aging mice. The states of the mice were recorded, the mice were sacrificed after one week, and organs such as livers and kidneys were immediately collected for subsequent detection. A cellular senescence β-galactosidase staining kit (Beyotime) was used. Frozen sections were fixed with a fixative for 15 minutes, rinsed 3 times with PBS, and incubated in an SA-β-gal solution at 37°C in the dark for 16-18 hours. The proportion of a stained area was quantitatively determined using ImageJ (NIH) software.

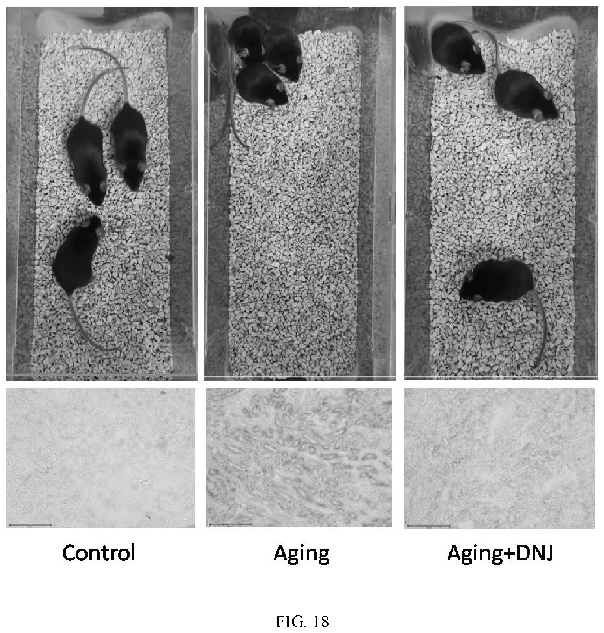

Results are shown in FIG. 18. Compared with the mice in the normal control group, the Dox-induced aging mice exhibit curled bodies, lethargic states, and significantly reduced motor abilities, and frozen section staining of organs such as kidneys shows that the number of SA-β-Gal staining-positive cells is significantly increased, presenting a senescence phenotype. In the aging mice treated with DNJ in the administration group, the physical states, motor abilities, and the like of the mice are all significantly improved, and the number of SA-β-Gal staining-positive cells in organs such as kidneys is significantly reduced, suggesting that DNJ or its derivatives have a good effect in delaying aging *in vivo*.

## Claims

1. Use of a 1-deoxynojirimycin compound in preparation of an anti-aging drug, wherein the 1-deoxynojirimycin compound is at least one of the following:
1-deoxynojirimycin or a pharmaceutically acceptable salt thereof; and
a 1-deoxynojirimycin derivative or a pharmaceutically acceptable salt thereof.

2. The use according to claim 1, wherein the 1-deoxynojirimycin derivative is any one of the following:
(1) DNJ-1, having the structure of formula II: 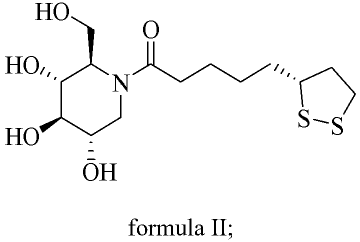
(2) the structure of general formula: 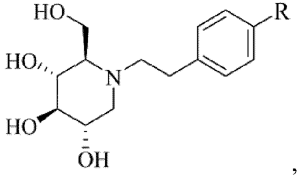 wherein R is selected from -H, -NO₂, and -X, wherein X is F, Cl, Br, or I; and
(3) DNJ-5a, having the structure of formula V:

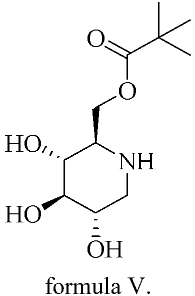

3. The use according to claim 2, wherein when R is selected as -H, the 1-deoxynojirimycin derivative is DNJ-3a, having the structure of formula III:

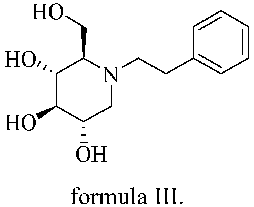

4. The use according to claim 2, wherein when R is selected as -X and X is F, the 1-deoxynojirimycin derivative is DNJ-3b, having the structure of formula IV:

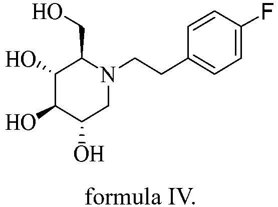

5. The use according to claim 2, wherein
(i) when the 1-deoxynojirimycin derivative is (1), compounds a and b1 are taken and allowed to undergo a condensation reaction in a solvent to obtain the corresponding 1-deoxynojirimycin derivative, wherein the compound a is 1-deoxynojirimycin, and the compound b1 is: 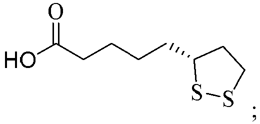
(ii) when the 1-deoxynojirimycin derivative is (4), compounds a and b4 are taken and allowed to undergo a condensation reaction in a solvent to obtain the corresponding 1-deoxynojirimycin derivative, wherein the compound a is 1-deoxynojirimycin, and the compound b4 is: 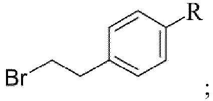 and
(iii) when the 1-deoxynojirimycin derivative is (2), 1-deoxynojirimycin first reacts with benzyl chloroformate to generate an intermediate product 1: 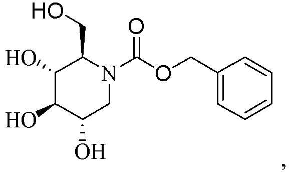
then, the intermediate product 1 reacts with PivCl to obtain an intermediate product 2: 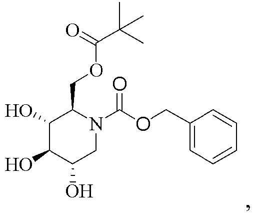
and finally, the intermediate product 2 is reduced to obtain the 1-deoxynojirimycin derivative.

6. The use according to claim 1, wherein an anti-aging effect of the anti-aging drug is prevention before the occurrence of aging, alleviation during the occurrence of aging, or repair after the occurrence of aging.

7. The use according to claim 1, wherein the anti-aging drug further comprises a pharmaceutically acceptable excipient.

8. The use according to claim 1, wherein a dosage form of the anti-aging drug is at least one of a suspension, a granule, a capsule, a powder, a tablet, an emulsion, a dropping pill, an injection, a suppository, an enema, an aerosol, a patch, or a drop.
